# FASCICULE DE BREVET EUROPEEN

(11) **EP 0 560 682 B1**
(45) Date de publication et mention de la délivrance du brevet: **07.08.1996**
(21) Numéro de dépôt: 93400624.8
(22) Date de dépôt: 11.03.1993
(51) Int. Cl.: C07D 209/12, A61K 7/13

(54) **Composition de teinture des fibres kératiniques à base de 4-monoindolyl 1,2-naphtoquinones , nouveaux composés 4-monoindolyl 1,2-naphtoquinones**
Färbemittel für keratinische Fasern, die 4-Monoindolyl 1,2-naphthochinone enthalten, 4-Monoindolyl 1,2-naphthochinone
Keratinous fibres dye compositions with 4-monoindolyl 1,2-naphthochinones, 4-monoindolyl 1,2-naphthoquinones

(30) Priorité: 13.03.1992 FR 9203048
(43) Date de publication de la demande: 15.09.1993
(73) Titulaire: L'OREAL, F-75008 Paris (FR)
(72) Inventeur: Henrion, Jean-Christophe, F-94160 Saint-Mandé (FR); Philippe, Michel, F-92160 Antony (FR); Hocquaux, Michel, F-75012 Paris (FR)
(74) Mandataire: Casalonga, Axel

(56) Documents cités:
- EP-A- 0 376 776
- EP-A- 0 460 996
- JOURNAL OF THE CHEMICAL SOCIETY Mars 1951, LONDON pages 703 - 712 JOHN D. BU'LOCK ET AL. 'Melanin and its Precursors. Part II.'
- JOURNAL OF THE CHEMICAL SOCIETY 1959, LONDON pages 2366 - 2375 J. MALCOLM BRUCE 'Heterocyclic compounds of Nitrogen. Part II.'

## Description

La présente invention concerne des compositions de teinture des fibres kératiniques, en particulier des cheveux humains, à base de 4-monoindolyl 1,2-naphtoquinones, ainsi que les nouveaux composés 4-monoindolyl 1,2-naphtoquinones.

On connaît dans l'état de la technique des produits de réaction d'addition de composés indoliques avec des quinones telles que la 1,2-naphtoquinone. Les synthèses de la 4-(3-indolyl)1,2-naphtoquinone et de la 4-(2-méthyl 3-indolyl)1,2-naphtoquinone ont été décrites dans l'article de BU'LOCK & HARLEY-MASON (J. Chem. Soc. 703, 1951).

La préparation de la 4-(3-indolyl)1,2-naphtoquinone a été également décrite dans l'article de BRUCE (J. Soc. Chem. 2366, 1959).

Les demandes EP-0376776 et 0460996 décrivent un procédé de teinture en deux étapes, mettant en oeuvre des mono- ou des dihydroxyindoles ou des aminoindoles qui sont oxydés, par des quinones, pour former un pigment mélanique, in situ dans les fibres kératiniques.

La demanderesse vient de découvrir d'une manière surprenante que des composés 4-monoindolyl 1,2-naphtoquinones pouvaient être utilisés dans la teinture directe des fibres kératiniques, en particulier des cheveux humains et conduire sans l'intermédiaire d'un système oxydant, à une large gamme de colorations possédant de nombreuses nuances à composante bleue.

La présente invention concerne donc des compositions de teinture directe des fibres kératiniques contenant des composés 4-monoindolyl 1,2- naphtoquinones.

Un autre objet concerne un procédé de teinture directe des fibres kératiniques utilisant ces compositions.

L'invention concerne également les nouveaux composés 4-monoindolyl 1,2-naphtoquinones. D'autres objets apparaîtront à la lumière de la description et des exemples qui suivent.

Les compositions de teinture des fibres kératiniques de l'invention sont caractérisées par le fait qu'elles contiennent dans un milieu approprié pour la teinture, au moins un composé répondant la formule suivante : 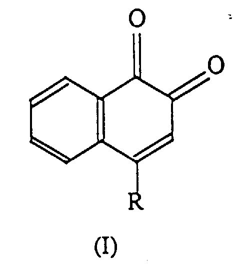 dans laquelle :
R désigne un groupement de formule (III) : 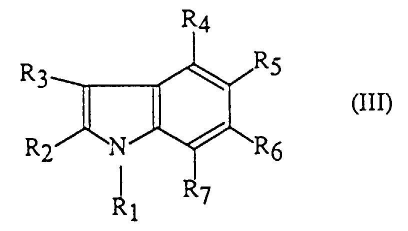 dans laquelle :
R₁ désigne un atome d'hydrogène, un radical alkyle en C₁-C₄ ou une liaison covalente;
R₂ désigne un atome d'hydrogène, un radical alkyle en C₁-C₄, phényle ou une liaison covalente;
R₃ désigne un radical alkyle en C₁-C₄ ou une liaison covalente;
un et un seul des radicaux R₁, R₂ et R₃ désigne une liaison covalente;
R₄ à R₇, identiques ou différents, désignent un atome d'hydrogène, un halogène, un radical alkyle en C₁-C₄, alcoxy(C₁-C₄) carbonyle, acyle en C₂C₄, acyl(C₂-C₄)oxy, cyano, amino éventuellement monosubstirué par un alkyle en C₁-C₄ ou un hydroxyalkyle en C₁-C₄, NO₂, OH, alcoxy(C₁-C₄), benzyloxy;
R₅ et R₆ peuvent former un cycle méthylènedioxy;
au plus un seul des radicaux R₄ à R₇ peut désigner halogène, NO₂, alcoxycarbonyle, acyle, cyano, amino éventuellement monosubstitué, ou acyloxy;
au plus deux des radicaux R₄ à R₇ peuvent désigner OH, ou alcoxy ou benzyloxy.

Les composés de formule (I) de l'invention sont nouveaux à l'exception des composés 4-(3-indolyl)1,2-naphtoquinone et 4-(2-méthyl 3-indolyl)1,2-naphtoquinone.

Les compositions de l'invention contiennent à titre préférentiel les composés suivants :
- la 4-(1-méthyl 3-indolyl)1,2-naphtoquinone,
- la 4-(3-indolyl)1,2-naphtoquinone,
- la 4-(2-méthyl 3-indolyl)1,2-naphtoquinone,
- la 4-(1,2-diméthyl 3-indolyl)1,2-naphtoquinone,
- la 4-(2-phényl 3-indolyl)1,2-naphtoquinone,
- la 4-(3-méthyl 2-indolyl)1,2-naphtoquinone,
- la 4-(3-méthyl 1-indolyl)1,2-naphtoquinone.

Les composés de formule (I) conformes à la présente invention peuvent être obtenus par condensation d'un composé indolique de formule (1): 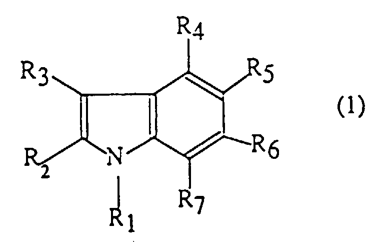 dans laquelle :
R₁ désigne l'hydrogène, un radical alkyle en C₁-C₄;
R₂ désigne l'hydrogène, un radical alkyle en C₁-C₄, phényle;
R₃ désigne l'hydrogène, un radical alkyle en C₁-C₄; au moins l'un des radicaux R₁, R₂ et R₃ désignant l'hydrogène;
R₄ à R₇ ont les mêmes significations indiquées ci-dessus, sur la 1,2-naphtoquinone, en présence d'un agent complexant de la quinone, d'un agent oxydant et du tertiobutanol.

On utilise de préférence le chlorure de Cerium III heptahydrate comme complexant de la quinone et l'iodate de sodium comme oxydant. La réaction est généralement achevée après 30 minutes d'agitation à 40°C.

Les composés 4-(3-indolyl)1,2-naphtoquinones de formule (I) (où le radical R₃ désigne une liaison covalente) sont obtenus et isolés du mélange brut par précipitation et cristallisation. Les rendements observés varient de 50 à 98%.

Les composés 4-(2-indolyl)1,2-napbtoquinones et 4-(1-indolyl) 1,2-naphtoquinones de formule (I) (où le radical R₂ ou R₁ désigne une liaison covalente) sont obtenus sous forme de mélange au cours de la même réaction. Ils sont séparés par chromatographie préparative sur colonne de gel de silice.

L'indolylnaphtoquinone est mise en solution dans l'éther éthylique sous argon, puis agitée en présence de 2 équivalents de dithionite de sodium en solution aqueuse à température ambiante, jusqu'à décoloration totale (12 heures). On décante, sèche la phase organique et évapore le solvant.

Les compositions de teinture conformes à la présente invention comprennent un milieu approprié pour la teinture constitué par de l'eau, un solvant ou un mélange d'eau et d'un ou plusieurs solvant(s) cosmétiquement acceptable(s).

Les solvants sont choisis plus particulièrement parmi les alcools inférieurs en C₁-C₆, les alkylèneglycols comme l'éthylèneglycol, le propylèneglycol, les éthers de glycol comme les éthers monométhylique, monoéthylique ou monobutylique de l'éthylèneglycol, l'acétate de monoéthyléther de l'éthylèneglycol, les monométhyléthers du propylèneglycol et du dipropylèneglycol; le lactate de méthyle.

Les solvants particulièrement préférés sont l'alcool éthylique et le propylèneglycol.

Les colorants de formules (I) sont présents dans des concentrations comprises de préférence entre 0,01 et 10% en poids et en particulier entre 0,05 et 5% en poids par rapport au poids total de la composition.

Les compositions conformes à l'invention peuvent également contenir d'autres colorants directs de structure différente de celle des composés de formule (I) et notamment des azoïques, des anthraquinoniques, des dérivés nitrés benzéniques et d'autres adjuvants habituellement utilisés en cosmétique, tels que les agents tensio-actifs anioniques, cationiques, non-ioniques, amphotères ou leurs mélanges, des épaississants, des parfums, des séquestrants, des agents filmogènes, des agents de traitement des fibres kératiniques, des agents dispersants, des agents de conditionnement des fibres kératiniques, des conservateurs, des agents de gonflement des fibres kératiniques.

Les compositions selon l'invention peuvent se présenter sous diverses formes, telles que des lotions plus ou moins épaissies ou gélifiées, des dispersions, des crèmes, des mousses, des émulsions. Elles peuvent notamment être conditionnées dans des dispositifs aérosols.

Un autre objet de l'invention concerne un procédé de teinture directe des fibres kératiniques, en particulier des cheveux humains, consistant à appliquer sur ces derniers une composition telle que définie précédemment, les cheveux étant éventuellement rincés puis séchés. Dans le cas où l'application est suivie d'un rinçage, la composition est laissée en contact avec les cheveux pendant 5 à 45 minutes et de préférence entre 10 et 30 minutes.

Les exemples qui suivent servent à illustrer l'invention sans toutefois présenter un caractère limitatif.

### EXEMPLE DE PREPARATION

Les spectres RMN ont été enregistrés sur des appareils BRUCKER WM250 ou W200, le tétraméthylsilane pris comme référence interne, le solvant étant le chloroforme deutérié ou le diméthylsulfoxyde hexadeutérié. Les spectres IR ont été recueillis sur un appareil PERKIN-ELMER 781 et les spectres UV sur un appareil CARY 118. Les spectres de masse ont été effectués sur un appareil AEI MS 30.

La 1,2-naphtoquinone et les indoles sont tous des composés commerciaux. Les indoles sont utilisés tels quels, et la naphtoquinone est purifiée par filtration sur silice.

### EXEMPLE 1

### 4-(3-indolyl)1,2-naphtoquinone (1).

Dans un ballon de 250 ml, on dissout à 40°C 2,00 g (12,65 mmoles) de 1,2-naphtoquinone purifiée, 4,71 g (12,65 mmoles) de chlorure de cérium III heptahydraté et 2,50 g (12,65 mmoles) d'iodate de sodium dans 50 ml de tertiobutanol. Puis on ajoute à la spatule 1,48 g (12,65 mmoles) d'indole et agite le mélange à cette température pendant 30 minutes. Puis on coule la solution sur 200 ml d'eau froide, filtre le précipité formé qui est lavé à l'eau, puis à l'acétone. Après cristallisation dans le tétrahydrofurane, on obtient 1,88 g (6,89 mmoles) de 4-(3-indolyl)1,2-naphtoquinone (1). Rdt. 54%. Après recristallisation dans le tétrahydrofurane, le produit fond à 258°C.

### EXEMPLE 2

### 4-(1-méthyl 3-indolyl)1,2-naphtoquinone (2).

En suivant le mode opératoire décrit à l'exemple 1, 2,00 g (12,65 mmoles) de 1,2-naphtoquinone, 4,71 g (1 eq.) de CeCl₃,7H₂O, 2,50 g (1 eq.) de NaIO₃ et 1,62 ml (1 eq.) de N-méthylindole dans 50 ml de tertiobutanol donnent 3,54 g (12,33 mmoles) de 4-(1-méthyl 3-indolyl)1,2-naphtoquinone (2). Rdt. 98 %. Après recristallisation dans le tétrahydrofurane, le produit fond à 218°C.

### EXEMPLE 3

### 4-(2-méthyl 3-indolyl)1,2-naphtoquinone (3).

En suivant le mode opératoire décrit à l'exemple 1, 10,00 g (63,23 mmoles) de 1,2-naphtoquinone, 23,56 g (1 eq.) de CeCl₃,7H₂O, 12,51 g (1 eq.) de NaIO₃ et 8,21 g (1 eq.) de 2-méthylindole dans 250 ml de tertiobutanol donnent 12,17 g (42,36 mmoles) de 4-(2-méthyl 3-indolyl)1,2-naphtoquinone (3). Rdt. 67%. Après recristallisation dans le tétrahydrofurane, le produit fond à 207°C.

### EXEMPLE 4

### 4(1,2-diméthyl 3-indolyl)1,2-naphtoquinone (4).

En suivant le mode opératoire décrit à l'exemple 1, 2,00 g ( 12,65 mmoles) de 1,2-naphtoquinone, 4,71 g (1 eq.) de CeCl₃,7H₂O, 2,50 g (1 eq.) de NaIO₃ et 1,84 g (1 eq.) de 1,2-diméthylindole dans 50 ml de tertiobutanol donnent 2,32 g (7,71 mmoles) de 4-(1,2-diméthyl 3-indolyl)1,2-naphtoquinone (4). Rdt. 62%. Après recristallisation dans le tétrahydrofurane, le produit fond à 207°C.

### EXEMPLE 5

### 4-(2-phényl 3-indolyl)1,2-naphtoquinone (5).

En suivant le mode opératoire décrit à l'exemple 1, 2,00 g (12,65 mmoles) de 1,2-naphtoquinone, 4,71 g (1 eq.) de CeCl₃,7H₂O, 2,50 g (1 eq.) de NaIO₃ et 2,44 g (1 eq.) de 2-phénylindole dans 50 ml de tertiobutanol donnent 3,60 g (10,32 mmoles) de 4-(2-phényl 3-indolyl)1,2-naphtoquinone (5). Rdt. 82%. Après recristallisation dans le tétrahydrofurane, le produit fond à 287°C.

### EXEMPLE 6

### 4-(3-méthyl 2-indolyl)1,2-naphtoquinone (6) et 4-(3-méthyl 1-indolyl)1,2-naphtoquinone (7).

En suivant le mode opératoire décrit à l'exemple 1, 2,00 g (12,65 mmoles) de 1,2-naphtoquinone, 4,71 g (1 eq.) de CeCl₃,7H₂O, 2,50 g (1 eq.) de NaIO₃ et 1,66 g (1 eq.) de 3-méthyindole dans 50 ml de tertiobutanol donnent, après chromatographie analytique sur colonne de gel de silice, 150 mg (0,52 mmole) de 4-(3-méthyl 2-indolyl)1,2-naphtoquinone bleue (6). P.F. 169°C et 100 mg (0,35 mmole) de 4-(3-méthyl 1-indolyl)1,2-naphtoquinone rouge (7). Après recristallisation dans le tétrahydrofurane, le produit fond à 169°C.

### Données spectroscopiques

Les tableaux suivants rendent compte des données spectroscopiques des diverses 4-indolyl 1,2-naphtoquinones synthétisées.

ε = coefficient d'extinction molaire.

**Tableau 1 :**

| UV Visible dans le tétrahydrofurane 99,8%. | |
|---|---|
| Composés | λmax (nm) , (Log₁₀ ε) |
| (6) | 249(4,40), 279(4,07), 286(3,04), 338(3,50), 379(3,56) 470(3,49) |
| (1) | 249(4,41), 285(4,00), 332(3,50), 380(3,53), 480(3,48) |
| (7) | 250(4,58), 280(3,98), 286(4,05), 336(3,54), 389(3,59) 484(3,59), 570(3,21) |
| (2) | 254(4,40), 286(4,07), 293(3,06), 336(3,41), 392(3,45) 494(3,47), 572(3,14) |
| (3) | 244(4,61), 304(4,37), 386(3,47), 490(3,41), 572(2,93) |
| (4) | 247(4,43), 287(3,92), 348(3,68), 363(3,69), 485(3,50) |
| (5) | 247(4,48), 284(3,88), 339(3,66), 362(3,61), 463(3,52) |

**Tableau 2:**

| Infra-rouge en pastilles de KBr. | | |
|---|---|---|
| Composés | γC=O (cm⁻¹) | γN-H (cm⁻¹) |
| (6) | 1630 | 3250 |
| (1) | 1630 | - |
| (7) | 1640 | 3270 |
| (2) | 1645 | - |
| (3) | 1640 | 3360 |
| (4) | 1635 | 3280 |
| (5) | 1645 | - |

**Tableau 3:**

| Spectrométrie de masse. | |
|---|---|
| Composés | m/z (%) |
| (6) | 275(50), 273(PM25), 245(100), 217(55), 189(15), 123(10), 109(15), 95(13) |
| (1) | 289(28), 287(PM30), 259(100), 246(16), 231(17), 229(29), 216(23), 202(10), 189(13), 115(11), 101(9) |
| (7) | 289(33), 287(PM40), 259(85), 230(100), 202(24), 189(18), 154(10), 114(18), 102(16) |
| (2) | 303(82), 301(PM78), 273(88), 272(70), 244(100), 218(43), 202(18), 121(20), 114(18), 101(13) |
| (3) | 351(82), 349(PM37), 320(86), 304(63), 291(58), 193(100), 146(61) |
| (4) | 289(50), 287(PM21), 256(27), 239(10), 230(26), 170(42), 130(28), 120(25), 105(28), 83(60), 71(35) |
| (5) | 289(100), 287(PM65), 272(32), 259(90), 244(15), 230(32), 130(35), 121(19), 114(18), 101(28) |

Les spectres RMN ¹H et ¹³C sont conformes à la structure attendue dans les exemples 1 à 6.

### EXEMPLE D'APPLICATION

On prépare la lotion colorante ayant la composition suivante:
- 4-(1-méthyl 3-indolyl)1,2-naphtoquinone 0,1 g
- Polyvinylpyrrolidone vendue sous la dénomination PVPK30 par la Société GAF 0,5 g
- Ethanol 40 g
- Eau qsp 100 g

Le pH est ajusté à 7,4 par de la triéthanolamine.

On applique cette lotion de mise en plis sur des cheveux décolorés. Après séchage, les cheveux sont colorés en blond cendré.

## Revendications

1. Composition de teinture des fibres kératiniques, en particulier des cheveux humains, caractérisée par le fait qu'elle contient dans un milieu approprié pour la teinture, au moins un composé répondant à la formule suivante : 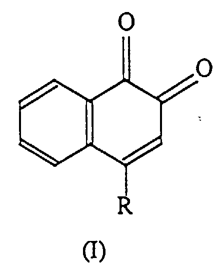 dans laquelle R désigne un groupement de formule : 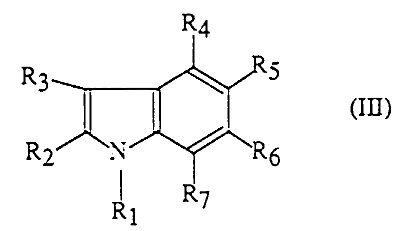 dans laquelle :
R₁ désigne un atome d'hydrogène, un radical alkyle en C₁-C₄ ou une liaison covalente;
R₂ désigne un atome d'hydrogène, un radical alkyle en C₁-C₄, phényle ou une liaison covalente;
R₃ désigne un radical alkyle en C₁-C₄ ou une liaison covalente;
un et un seul des radicaux R₁, R₂ et R₃ désigne une liaison covalente;
R₄ à R₇, identiques ou différents, désignent un atonie d'hydrogène, un halogène, un radical alkyle en C₁-C₄, alcoxy(C₁-C₄) carbonyle, acyle en C₂-C₄, acyl(C₂-C₄)oxy, cyano, amino éventuellement monosubstitué par un alkyle en C₁-C₄ ou un hydroxyalkyle en C₁-C₄, NO₂, OH, benzyloxy, alcoxy(C₁-C₄);
R₅ et R₆ peuvent former un cycle méthylènedioxy;
au plus un seul des radicaux R₄ à R₇ peut désigner halogène, NO₂, alcoxycarbonyle, acyle, cyano, amino éventuellement substitué, ou acyloxy;
au plus deux des radicaux R₄ à R₇ peuvent désigner OH, alcoxy ou benzyloxy.

2. Composition selon la revendication 1, caractérisée par le fait que les composés de formules (I) sont choisis parmi :
- la 4-(1-méthyl 3-indolyl)1,2-naphtoquinone,
- la 4-(3-indolyl)1,2-naphtoquinone,
- la 4-(2-méthyl 3-indolyl)1,2-naphtoquinone,
- la 4-(1,2-diméthyl 3-indolyl)1,2-naphtoquinone,
- la 4-(2-phényl 3-indolyl)1,2-naphtoquinone,
- la 4-(3-méthyl 2-indolyl)1,2-naphtoquinone,
- la 4-(3-méthyl 1-indolyl)1,2-naphtoquinone.

3. Composition selon la revendication 1 ou 2, caractérisée par le fait que le composé de formule (I) est présent à des concentrations comprises entre 0,01 et 10% en poids par rapport au poids total de la composition.

4. Composition selon l'une quelconque des revendications 1 à 3, caractérisée par le fait que le milieu approprié pour la teinture est constitué d'eau, de solvant ou d'un mélange d'eau et d'un ou plusieurs solvants choisis parmi les alcools inférieurs en C₁-C₆, les alkylène glycols, les éthers de glycol comme les éthers monométhylique, monoéthylique ou monobutylique de l'éthylèneglycol, l'acétate du monoéthyléther de l'éthylèneglycol, les monométhyléthers du propylèneglycol et du dipropylèneglycol, le lactate de méthyle.

5. Composition selon l'une quelconque des revendications 1 à 4, caractérisée par le fait qu'elle contient en plus des colorants directs autres que ceux de formule (I), des agents tensio-actifs anioniques, cationiques, non-ioniques, amphotères ou leurs mélanges, des épaississants, des parfums, des séquestrants, des agents filmogènes, des agents de traitement des fibres kératiniques, des agents dispersants, des agents de conditionnement des fibres kératiniques, des conservateurs, des agents de gonflement des fibres kératiniques.

6. Composition selon l'une quelconque des revendications 1 à 5, caractérisée par le fait qu'elle se présente sous forme de lotion plus ou moins épaissie ou gélifiée, de dispersion, de crème, de mousse, ou conditionnée dans un dispositif aérosol.

7. Composé répondant à la formule suivante : 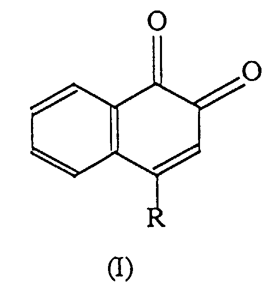 dans laquelle R désigne un groupement de formule : 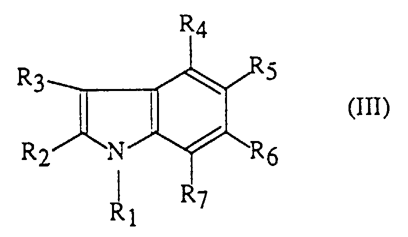 dans laquelle :
R₁ désigne un atome d'hydrogène, un radical alkyle en C₁-C₄ ou une liaison covalente;
R₂ désigne un atome d'hydrogène, un radical alkyle en C₁-C₄, phényle ou une liaison covalente;
R₃ désigne un radical alkyle en C₁-C₄ ou une liaison covalente;
un et un seul des radicaux R₁, R₂ et R₃ désigne une liaison covalente;
R₄ à R₇, identiques ou différents, désignent un atome d'hydrogène, un halogène, un radical alkyle en C₁-C₄, alcoxy(C₁-C₄) carbonyle, acyle en C₂-C₄, acyl(C₂-C₄)oxy, cyano, amino, NO₂, OH, benzyloxy, alcoxy(C₁-C₄);
R₅ et R₆ peuvent former un cycle méthylènedioxy;
au plus un seul des radicaux R₄ à R₇ peut désigner halogène, NO₂, alcoxycarbonyle, acyle, cyano, amino ou acyloxy;
au plus deux des radicaux R₄ à R₇ peuvent désigner OH, benzyloxy ou alcoxy;
sous réserve que :
lorsque R₁ et les radicaux R₄ à R₇ désignent l'hydrogène, R₂ ne désigne pas de radical méthyle ou l'hydrogène.

8. Procédé de teinture directe des fibres kératiniques, en particulier des cheveux humains, caractérisé par le fait qu'on applique sur les fibres au moins une composition selon l'une quelconque des revendications 1 à 6, l'application étant éventuellement suivie d'un rinçage.

## Patentansprüche

1. Zusamensetzung zur Färbung keratinischer Fasern, insbesondere menschlicher Haare,
dadurch **gekennzeichnet**, daß
sie in einem zur Färbung geeigneten Milieu mindestens eine Verbindung der folgenden Formel enthält: 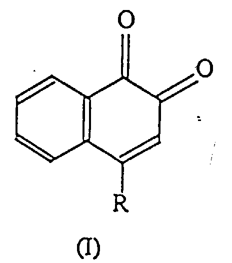 worin gilt:
R bedeutet eine Gruppierung der Formel (III): 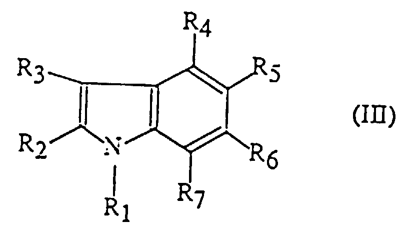 worin gilt:
R₁ bedeutet ein Wasserstoffatom, einen C₁₋₄-Alkylrest oder eine kovalente Bindung;
R₂ bedeutet ein Wasserstoffatom, einen C₁₋₄-Alkylrest, einen Phenylrest oder eine kovalente Bindung;
R₃ bedeutet einen C₁₋₄-Alkylrest oder eine kovalente Bindung; einer und nur einer der Reste R₁, R₂ und R₃ bedeuten eine kovalente Bindung;
R₄ bis R₇ bedeuten, gleich oder verschieden, ein Wasserstoffatom, Halogen, einen C₁-₄-Alkyl-, C₁₋₄-Alkoxycarbonyl-, C₂₋₄-Acyl-, C₂₋₄-Acyloxy-, Cyano-, einen gegebenenfalls mit einem C₁₋₄-Alkyl- oder Hydroxy-C₁₋₄-alkylrest monosubstituierten Amino-, einen NO₂-, OH-, C₁₋₄-Alkoxy- oder einen Benzyloxyrest;
R₅ und R₆ können auch einen Methylendioxy-Ring bilden;
höchstens einer der Reste R₄ bis R₇ kann Halogen, einen NO₂-, Alkoxycarbonyl-, Acyl-, Cyano-, einen gegebenenfalls monosubstituierten Amino- oder einen Acyloxyrest bedeuten;
höchstens zwei der Reste R₄ bis R₇ können OH oder Alkoxy-oder Benzyloxyreste bedeuten.

2. Zusammensetzung nach Anspruch 1,
dadurch **gekennzeichnet**, daß
die Verbindungen der Formel (I) ausgewählt sind aus:
- 4-(1-Methyl-3-indolyl)-1,2-naphthochinon,
- 4-(3-Indolyl)-1,2-natphthochinon,
- 4-(2-Methyl-3-indolyl)-1,2-naphthochinon,
- 4-(1,2-Dimethyl-3-indolyl)-1,2-naphthochinon,
- 4-(2-Phenyl-3-indolyl)-1,2-naphthochinon,
- 4-(3-Methyl-2-indolyl)-1,2-naphthochinon,
- 4-(3-Methyl-l-indolyl)-1,2-naphthochinon.

3. Zusamensetzung gemäß Anspruch 1 oder 2,
dadurch **gekennzeichnet**, daß
die Verbindung der Formel (I) in Konzentrationen von 0,01 bis 10 Gew.% vorhanden ist, bezogen auf das Gesamtgewicht der Zusammensetzung.

4. Zusammensetzung gemäß einem der Ansprüche 1 bis 3,
dadurch **gekennzeichnet**, daß
das zur Färbung geeignete Milieu aus Wasser, einem Lösungsmittel oder einer Mischung aus Wasser und einem oder mehreren Lösungsmitteln zusammengesetzt ist, die aus C₁₋₆-Niedrigalkoholen, Alkylenglycolen, Glycolethern wie den Monomethyl-, Monoethyl- oder Monobutylethern von Ethylenglycol, Ethylenglycolmonoethyletheracetat, den Monomethylethern von Propylenglycol und Dipropylenglycol sowie aus Methyllactat ausgewählt sind.

5. Zusammensetzung gemäß einem der Ansprüche 1 bis 4,
dadurch **gekennzeichnet**, daß
sie ausserdem Direktfarbstoffe, die sich von denjenigen der Formel (I) unterscheiden, anionische, kationische, nichtionische oder amphotere oberflächenaktive Mittel oder deren Mischungen, Verdickungsmittel, Parfüm-Produkte, Sequestriermittel, filmbildende Mittel, Mittel zur Behandlung der keratinischen Fasern, Dispergiermittel, Mittel zum Konditionieren der keratinischen Fasern, Konservierungsstoffe sowie Mittel zur Auflockerung der keratinischen Fasern enthält.

6. Zusammensetzung gemäß einem der Ansprüche 1 bis 5,
dadurch **gekennzeichnet**, daß
sie in Form einer mehr oder weniger verdickten oder gelierten Lotion, einer Dispersion, Creme oder eines Schaums oder in einer Aerosol-Vorrichtung zubereitet vorliegt.

7. Verbindung gemäß der folgenden Formel: 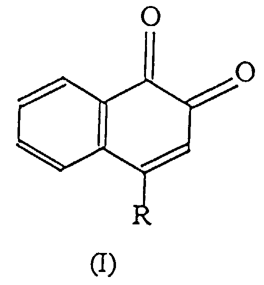 worin R eine Gruppierung der Formel bedeutet: 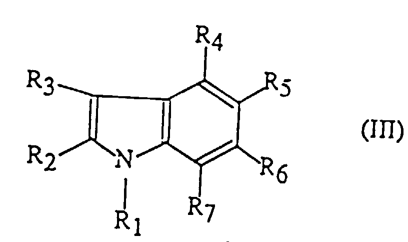 worin gilt:
R₁ bedeutet ein Wasserstoffatom, einen C₁₋₄-Alkylrest oder eine kovalente Bindung;
R₂ bedeutet ein Wasserstoffatom, einen C₁₋₄-Alkylrest, einen Phenylrest oder eine kovalente Bindung;
R₃ bedeutet einen C₁₋₄-Alkylrest oder eine kovalente Bindung;
nur einer der Reste R₁, R₂ und R₃ bedeutet eine kovalente Bindung;
R₄ bis R₇ bedeuten, gleich oder verschieden, ein Wasserstoffatom, Halogen, einen C₁₋₄-Alkyl-, C₁₋₄-Alkoxycarbonyl-, C₂₋₄-Acyl-, C₂₋₄-Acyloxy-, Cyano-, Amino-, NO₂-, OH-, C₁₋₄-Alkoxy- oder einen Benzyloxyrest;
R₅ und R₆ können einen Methylendioxy-Ring bilden;
höchstens einer der Reste R₄ bis R₇ kann Halogen, einen NO₂-, Alkoxycarbonyl-, Acyl-, Cyano-, Amino- oder einen Acyloxyrest bedeuten;
höchstens zwei der Reste R₄ bis R₇ können OH, einen Benzyloxy oder Alkoxyrest bedeuten,
mit der Maßgabe, daß gilt:
wenn R₁ und die Reste R₄ bis R₇ Wasserstoff bedeuten, R₂ einen Methylrest oder Wasserstoff nicht bedeutet.

8. Verfahren zur Direktfärbung keratinischer Fasern, insbesondere menschlicher Haare,
dadurch **gekennzeichnet**, daß
man auf die Fasern mindestens eine Zusammensetzung gemäß einem der Ansprüche 1 bis 6 aufbringt, worauf gegebenenfalls gespült wird.

## Claims

1. Composition for dyeing keratinous fibres, in particular human hair, characterized in that it contains, in a medium appropriate for dyeing, at least one compound corresponding to the following formula: 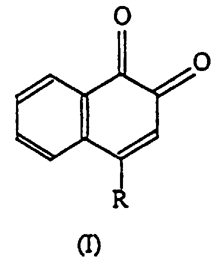 in which R denotes a group of formula: 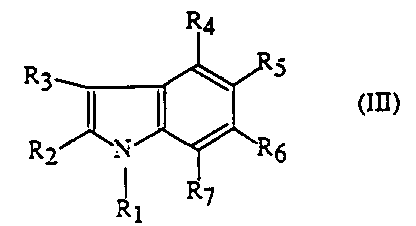 in which:
R₁ denotes a hydrogen atom, a C₁-C₄ alkyl radical or a covalent bond;
R₂ denotes a hydrogen atom, a C₁-C₄ alkyl or phenyl radical or a covalent bond;
R₃ denotes a C₁-C₄ alkyl radical or a covalent bond;
one and one alone of the R₁, R₂ and R₃ radicals denotes a covalent bond;
R₄ to R₇, which are identical or different, denote a hydrogen atom, a halogen or a C₁-C₄ alkyl, (C₁-C₄)alkoxycarbonyl, C₂-C₄ acyl, (C₂-C₄)acyloxy, cyano, amino, optionally monosubstituted by a C₁-C₄ alkyl or a C₁-C₄ hydroxyalkyl, NO₂, OH, benzyloxy or (C₁-C₄)alkoxy radical;
R₅ and R₆ can form a methylenedioxy ring;
at most one alone of the R₄ to R₇ radicals can denote halogen, NO₂, alkoxycarbonyl, acyl, cyano, optionally substituted amino or acyloxy;
at most two of the R₄ to R₇ radicals can denote OH, alkoxy or benzyloxy.

2. Composition according to Claim 1, characterized in that the compounds of formulae [sic] (I) are chosen from:
- 4-(1-methyl-3-indolyl) -1,2-naphthoquinone,
- 4-(3-indolyl)-1,2-naphthoquinone,
- 4-(2-methyl-3-indolyl)-1,2-naphthoquinone,
- 4-(1,2-dimethyl-3-indolyl)-1,2-naphthoquinone,
- 4-(2-phenyl-3-indolyl)-1,2-naphthoquinone,
- 4-(3-methyl-2-indolyl)-1,2-naphthoquinone,
- 4-(3-methyl-1-indolyl)-1,2-naphthoquinone.

3. Composition according to Claim 1 or 2, characterized in that the compound of formula (I) is present at concentrations of between 0.01 and 10 % by weight with respect to the total weight of the composition.

4. Composition according to any one of Claims 1 to 3, characterized in that the medium appropriate for dyeing is composed of water, of solvent or of a mixture of water and of one or a number of solvents chosen from lower C₁-C₆ alcohols, alkylene glycols, glycol ethers, such as the monomethyl, monoethyl or monobutyl ethers of ethylene glycol, the acetate of the monoethyl ether of ethylene glycol or the monomethyl ethers of propylene glycol and of dipropylene glycol, or methyl lactate.

5. Composition according to any one of Claims 1 to 4, characterized in that it additionally contains direct dyes other than those of formula (I), anionic, cationic, nonionic or amphoteric surface-active agents or their mixtures, thickeners, fragrances, sequestrants, film-forming agents, treatment agents for keratinous fibres, dispersing agents, conditioning agents for keratinous fibres, preservatives or swelling agents for keratinous fibres.

6. Composition according to any one of Claims 1 to 5, characterized in that it is provided in the form of a lotion, which is more or less thickened or gelled, a dispersion, a cream or a foam or packaged in an aerosol device.

7. Compound corresponding to the following formula: 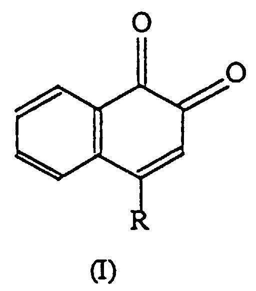 in which R denotes a group of formula: 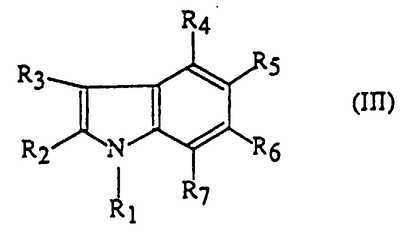 in which:
R₁ denotes a hydrogen atom, a C₁-C₄ alkyl radical or a covalent bond;
R₂ denotes a hydrogen atom, a C₁-C₄ alkyl or phenyl radical or a covalent bond;
R₃ denotes a C₁-C₄ alkyl radical or a covalent bond;
one and one alone of the R₁, R₂ and R₃ radicals denotes a covalent bond;
R₄ to R₇, which are identical or different, denote a hydrogen atom, a halogen or a C₁-C₄ alkyl, (C₁-C₄)alkoxycarbonyl, C₂-C₄ acyl, (C₂-C₄)acyloxy, cyano, amino, NO₂, OH, benzyloxy or (C₁-C₄)alkoxy radical;
R₅ and R₆ can form a methylenedioxy ring;
at most one alone of the R₄ to R₇ radicals can denote halogen, NO₂, alkoxycarbonyl, acyl, cyano, amino or acyloxy;
at most two of the R₄ to R₇ radicals can denote OH, benzyloxy or alkoxy;
provided that:
when R₁ and the R₄ to R₇ radicals denote hydrogen, R₂ does not denote methyl radical or hydrogen.

8. Process for the direct dyeing of keratinous fibres, in particular of human hair, characterized in that at least one composition according to any one of Claims 1 to 6 is applied to the fibres, the application optionally being followed by a rinsing.
